# EUROPEAN PATENT APPLICATION

(11) **EP 3 593 819 A1**
(43) Date of publication of application: **15.01.2020**
(21) Application number: 18305927.8
(22) Date of filing: 10.07.2018
(51) Int. Cl.: A61K 47/12, A61K 47/18, A61K 9/00, A61K 9/10, A61K 47/38, A61K 47/40, A61P 25/00, A61K 31/198, A61K 31/4045

(54) **COMPOSITIONS FOR THERAPEUTIC USES CONTAINING 5-HTP AND CARBIDOPA**

(71) Applicant: Medday Pharmaceuticals, 75008 Paris (FR)
(72) Inventor: The designation of the inventor has not yet been filed
(74) Representative: Flesselles, Bruno F.G.

(57) **Abstract**

The invention relates to a powder composition containing 5-HTP and carbidopa and its use for obtaining a suspension solution for use for treating various diseases and disorders.

## Description

The invention relates to the field of pharmaceutical compositions and to the treatment of diseases where lack of serotonin in the central nervous system is involved.

L-5-hydroxytryptophan (5-HTP) is a naturally occurring amino acid and a direct precursor of serotonin (5-TH). It is readily converted into serotonin in vivo without biochemical feedback. The development of 5-HTP as a therapeutic has been limited by its rapid degradation. Carbidopa (CBD) is a levodopa analogue and an inhibitor of decarboxylase (e.g., aromatic L-amino acid decarboxylase). Carbidopa prevents the peripheral decarboxylation of 5-HTP to 5-HT, which has a very short half-life. Carbidopa cannot pass across the blood-brain barrier, allowing more 5-HTP to be available to enter the brain, and less peripheral 5-HT to be formed, which causes systemic side effects. Indeed, simultaneous intake of carbidopa and 5-HTP increases blood levels of 5-HTP (Psychiatry. Res. 1982 Dec; 7 (3): 373-85, Kinetics of I-5-hydroxytryptophan in healthy subjects, Westenberg HG, Gerritsen TW, Meijer BA, van Praag HM). However, the use of 5-HTP has been limited by a small therapeutic window between the occurrence of neuroendocrine endpoints and the incidence of side effects. Therefore, there is a need to develop new formulations that allow for effective dosing of 5-HTP and carbidopa. These formulations should also have appropriate shelf-life and an acceptable palatability.

### Definitions

The term "5-HTP" refers to L-5-hydroxytryptophan, which is a naturally occurring amino acid and a direct precursor of serotonin (5-TH). It is readily converted into serotonin *in vivo.*

The term "active ingredient" refers to an ingredient in a pharmaceutical composition that is biologically active. Active ingredients in the context of this application are 5-HTP and carbidopa.

The term "autism spectrum disorders" refers to neurological and developmental disorders that affect communication and behavior. Examples of autism spectrum disorder include, e.g., autism, Asperger syndrome, pervasive developmental disorder not otherwise specified (PDD-NOS), and childhood disintegrative disorder.

The term "bulking agent" refers to an agent used to increase the volume of a pharmaceutical composition. Exemplary bulking agents include, but are not limited to, lactose, mannitol, sucrose, and mixture thereof.

The term "carbidopa" or "CBD" refers to a levodopa analogue and an inhibitor of decarboxylase (e.g., aromatic L-amino acid decarboxylase). Carbidopa prevents the peripheral decarboxylation of 5-HTP to 5-HT, which has a very short half-life. Carbidopa cannot pass across the blood-brain barrier, allowing more 5-HTP to be available to enter the brain, and less peripheral 5-HT to be formed, which can cause systemic side effects.

The term an "effective amount" of an agent, as used herein, is the amount sufficient to effect beneficial or desired results, such as clinical results, and an "effective amount" depends upon the context in which it is being applied. In the context of administering 5-HTP and carbidopa, an effective amount of an agent is, for example, an amount sufficient to achieve a reduction in the severity of a symptom of an autism spectrum disorder as compared to the response obtained without administration of the agent. An effective amount is an amount that provides therapeutic improvement while minimizing side or adverse effect.

The term "medicament" refers to an agent used for medical treatment.

The term "pharmaceutical composition" is a composition having pharmacological activity or other direct effect in the mitigation, treatment, or prevention of disease or disorder, and/or a finished dosage form or formulation thereof, and which is indicated for human use. In the context of the application, a pharmaceutical composition includes an active ingredient, such as 5-HTP and carbidopa. It may optionally include a thickener, bulking agent, a chelating agent and/or preservative.

The term "suspension formulation" refers a liquid formulation including solid particles disposed within a carrier liquid, e.g., a buffer, in which they are not soluble on an appropriate timescale.

The present invention provides new formulations containing 5-HTP and carbidopa, which are suitable for the treatment of diseases or disorders such as, for example, pervasive developmental disorders, schizophrenia, psychotic disorders (which may be associated with organic genetic diseases (e.g., organic psychoses)), hereditary metabolic diseases or neurogenetic diseases (e.g., mitochondrial diseases), certain acquired diseases (e.g., ponto-cerebellar hypoplases), Rett syndrome, epileptic encephalopathies, leukodystrophies, neuro-psychiatric disorders including autistic spectrum disorders and behavioral disorders, post-anoxic myoclonies, "Lance and Adams syndrome," cerebellar ataxias, depression, suicidal thoughts, anxiety, alcoholism, fibromyalgia, insomnia, bulimia, and chronic headache. Specific diseases are disorders of neurotransmitter metabolism and transport (Orphanet ORPHA:79169), and in particular disorders of biogenic amine metabolism and transport (Orphanet, ORPHA:79214) such as brain dopamine-serotonin vesicular transport disease. Of particular interest are tetrahydrobiopterin deficiencies (BH4 deficiencies) or any trouble of BH4 synthesis. One can thus cite guanosine triphosphate cyclohydrolase I (GTPCH) deficiency, 6-pyruvoyl tetrahydropterin synthase (PTPS) deficiency, pterin-4-alpha-carbinolamine dehydratase (PCD) deficiency and dihydropteridine reductase (DHPR) deficiency.

The structural formulas, chemical names, molecular formulas, and molar masses for 5-HTP and carbidopa are shown below.

| | 5-HTP | Carbidopa |
|---|---|---|
| Structural formula | 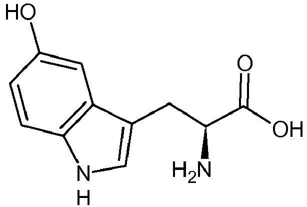 | 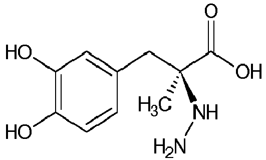 |
| Chemical name | (2*S*)-2-amino-3-(5-hydroxy-1*H*-indol-3-yl)propanoic acid | (2*S*)-3-(3,4-dihydroxyphenyl)-2-hydrazinyl-2-methylpropanoic acid |
| INN name | 5-hydroxytryptophan | Carbidopa |
| CAS number | 56-69-9 | 38821-49-7 |
| Molecular formula | C₁₁H₁₂N₂O₃ | C₁₀H₁₄N₂O₄ |
| Molar mass | 220.23 g/mol | 226.23 G/MOL |

Daily doses of 5-HTP are generally comprised between 50 and 250 mg, although it could be effective for doses as low as 10 or 25 mg. For some patients, higher doses may be needed (between 300-500 mg).

The use of 5-HTP has been limited by a small therapeutic window between the occurrence of neuroendocrine endpoints and the incidence of side effects. Side effects associated with 5-HTP have been reported to include, for example, heartburn, stomach pain, nausea, vomiting, diarrhea, drowsiness, sexual problems, and muscle problems. A rare but major adverse event potentially related to 5-HTP treatment is serotonin syndrome, which can be characterized by agitation, confusion, delirium, tachycardia, diaphoresis, and blood pressure fluctuations (Altern. Med. Rev., 1998;3:271-80. 5-Hydroxytryptophan: a clinically-effective serotonin precursor Birdsall, TC).

Therefore, since carbidopa allows for an increased blood concentration of 5-HTP, it is advantageous to use these two compounds to maintain as low as possible dose of 5-HTP for which a therapeutic effect is obtained (J. Clin. Psychopharmacol. 2002 Apr;22(2): 183-9. Placebo-controlled comparison of three dose-regimens of 5-hydroxytryptophan challenge test in healthy volunteers. Gijsman HJ1, van Gerven JM, de Kam ML, Schoemaker RC, Pieters MS, Weemaes M, de Rijk R, van der Post J, Cohen AF).

FR 2654931 discloses a solid composition including both 5-HTP and carbidopa. The tablets or capsule disclosed therein contain 50, 100, or 200 mg of 5-HTP, and 5, 10 or 20 mg of carbidopa, respectively, (10:1 (w/w) ratio). These formulations, however, do not allow the patient to finely adjust the daily dosage to obtain the therapeutic effect, while also minimizing the side effects.

US 6207699 discloses formulations for decreasing body weight including 5-HTP, carbidopa, and pindolol. This patent indicates that the formulation of the product could be liquid or a suspension formulation, however, it does not provide any further information regarding such formulations, only exemplifying capsules.

There is a need to develop new formulations that can allow the patient to precisely dose the amount of the composition 5-HTP / carbidopa that is taken. These formulations should also be stable for some time and have an acceptable palatability.

Since solid compositions do not allow for precise dosing, the Applicant has investigated the development of liquid formulations. However, and despite the teachings of US 6207699, the development of such formulations has proven to be challenging.

Indeed, the solubility of both 5-HTP and carbidopa was not sufficient to allow for a true solution of the mixture. Applicant also showed that 5-HTP is fairly stable in solution but that carbidopa is less stable. It is, however, important to obtain a formulation that would be stable for at least one week at patient's home. Consequently,

Applicant also tried to develop a two compartments product including a preserved viscous liquid in which the active ingredients carbidopa and 5-HTP would be dispersed at the time of patient delivery. This proved to be unsuccessful, as the liquid could only be efficiently preserved with methyl and propyl-parahydoxybenzoate which are toxic on repeated exposure and, thus, not suitable for prolonged treatment. Moreover, the taste was not palatable.

Applicant further tested a liquid formulation using a combination of sorbate and benzoate as preservatives. Unfortunately, sorbate is not stable in solution, and, therefore, degrades, and also leads to degradation of the thickener, causing a yellowish discoloration. In addition, it was also noted that the degraded sorbate promoted the degradation of carbidopa, which by itself also degrades. Parabens were tested as preservatives, and provided poor palatability, and were thereby not selected.

Following extensive research, Applicant describes herein a dosable formulation including both 5-HTP and carbidopa, where the two products are present together as a powder (i.e., pulverulent) formulation, with a solvent that is kept apart, and added at the time of use. The resulting suspension solution is stable for at least one week (e.g., two weeks, four weeks, two months, three months, four months, five months, six months, or a year) in the refrigerator (2 to 8°C). The taste of the formulation is also acceptable for a patient to take it daily.

In a first embodiment, the invention relates to a composition including 5-HTP and carbidopa, where the composition is in the form of a powder. This formulation is thus in a dry form. 5-HTP and carbidopa may be used in the form of the free base, a salt, solvate, or prodrug. The formulation is of pharmaceutical grade as it is intended for use as a medicine. In some embodiments, the amount of contaminants (i.e., components other than the active 5-HTP and carbidopa, degradation products thereof, and/or excipients) is less than 0.2% more preferably less than 0.1% (in particular less than 0.07%, 0.05%, 0.02%, or 0.01%). The formulation is thus in a form of a pharmaceutical powder blend that includes 5-HTP and carbidopa. In the preferred embodiment, the ratio of 5-HTP to carbidopa is between 5:1 and 1:1 (by weight) (e.g., 5:1, 4:1, 3:1, 2:1, or 1:1), preferably between 5:1 and 3:1, more preferably about 4:1, or exactly 4:1 ("about" meaning the value ±10%). This ratio has proven to be the most effective to obtain both stability of the product and synergistic effect of the two compounds.

In a specific embodiment, the powdered composition further includes a preservative, in particular sodium benzoate. It is preferred when the composition contains only 5-HTP, carbidopa, a preservative (preferably sodium benzoate). However, it may also include a bulking agent. In some embodiments, one shall introduce the preservative in the liquid solution that is to be added to the powdered solution for reconstitution of the suspension composition.

In a preferred embodiment, the powdered composition further includes a bulking agent. Indeed, in order to avoid the formation of lumps when mixing the solvent to the powder of 5-HTP and carbidopa, it may be useful to add a bulking agent in the powder container. The bulking agent can be selected from classical agents in the art, based on the reconstitution behavior and the stability of the reconstituted product. Bulking agent suitable for use in the composition include, but are not limited to, lactose, mannitol, sucrose and mixture thereof, in particular mixtures of lactose / sucrose or of mannitol / sucrose. However, it is preferred, if possible, to avoid the use of mannitol or lactose (for dietary requirements and for stability reasons). Consequently, sucrose is the preferred bulking agent used in the composition according to the invention. The amount of bulking agent in the first container is preferably comprised (as compared to the amount of both 5-HTP and carbidopa) between 1:1 and 8:1 (w/w) and is preferably 5:1, or 3:1 (3 parts of the bulking agent for 1 part of active molecules (5-HTP and carbidopa)). Using a quantity as low as possible is preferred in order to reduce the intake in rapid sugar while maintaining the ability to obtain a non-sticky composition.

Consequently, in a preferred embodiment, the powdered composition contains only the active ingredients (5-HTP, carbidopa) and the bulking agent.

It is also possible to add one or more desiccants to the powdered composition to ensure that it is maintained it in the dry form.

Then invention also relates to a suspension solution including 5-HTP and carbidopa in solution, in an acidic buffer (so that the suspension solution pH is about 3.0), in particular and preferably Citrate buffer. Citrate buffer can be made with citric acid monohydrate, C₆H₈0₇ • H₂O, with a molar mass of 210.14 g (0.1M-solution contains 21.01 g/L). pH (measured at a temperature of from about 20°C to about 30°C) can be adjusted with any basic solution such as NaOH, if necessary.

In this suspension solution, 5-HTP shall generally be in a soluble form (solubilized) and carbidopa shall be found in the form of particles in suspension. This suspension is for pharmaceutical use, in a patient, e.g., a human being.

It is preferred when the concentration of 5-HTP in the solution is comprised between 10 and 100 g/L, more preferably between 20 and 60 g/L, more preferably between 30 and 50 mg/mL. In particular, the concentration of 5-HTP in the suspension formulation is about or exactly 40 mg/mL.

It is preferred when the concentration of carbidopa in the solution is comprised between 2,5 and 25 g/L, more preferably between 5 and 20 g/L, more preferably between 5 and 15 mg/mL. In particular, the concentration of carbidopa in the suspension formulation is about or exactly 10 mg/mL.

Consequently, when the ratio 5-HTP:carbidopa is 4:1 (by weight) in the powdered formulation and the preferred concentration as disclosed above are to be obtained, one will add 20 mL of buffer to 1 g of such powdered solution.

It is preferred when the buffer used is citrate buffer 50mM with a pH lower than 4.5, preferably lower than 4, preferably lower than 3.5. The most preferred buffer in which to suspend the powdered formulation is citrate buffer 50mM, pH 3.0. As an alternative, one can use a solution with a pH 3.5.

The suspension formulation herein disclosed can thus be obtained (and is thus susceptible to be obtained) by addition of such citrate buffer to the powdered composition as disclosed above. Preferably, the relative quantities (buffer / powder) are chosen so as to obtain the concentrations as disclosed above.

The invention also relates to a method for preparing a suspension solution of carbidopa and 5-HTP, comprising the step of adding Citrate buffer (preferably as disclosed above and in particular 50mM at pH 3.0) to the powder composition as disclosed above. This makes it possible to obtain a suspension composition as disclosed. Shaking the mixture is preferred when preparing the solution or before use, in order to make sure that the 5-HTP dissolves or that the suspension is homogenous. In particular, the amount of buffer added to the powder composition is selected to obtain a concentration between 10 and 100 g/L of 5-HTP in the suspension composition, more preferably between 20 and 60 g/L, more preferably between 30 and 50 mg/mL. A concentration of 40 mg/mL of 5-HTP is particularly preferred.

The invention also relates to the suspension composition as disclosed as a medicament. It also relates to the suspension composition as disclosed for use as a medicament.

It also relates to the suspension composition as disclosed for its use in the treatment of a disease or disorder where increase of serotonin is required.

In particular, the disease or disorder is selected from the group consisting of pervasive developmental disorders, schizophrenia, psychotic disorders (which lay be associated with organic genetic diseases (such as organic psychoses) or not), hereditary metabolic diseases or neurogenetic diseases (such as mitochondrial diseases), certain acquired diseases (such as ponto-cerebellar hypoplases), Rett syndrome, epileptic encephalopathies, leukodystrophies, neuro-psychiatric disorders including autistic spectrum disorders and behavioural disorders, post-anoxic mycolonies, "Lance and Adams syndrome", cerebellar ataxias, depression, suicidal thoughts, anxiety, alcoholism, fibromyalgia, insomnia, bulimia, and chronic headache. All these diseases are known to require an increase of serotonin to alleviate these syndromes and the symptoms of the patient.

In particular, the formulation is particularly adapted to and may be used for the treatment of an autism spectrum disorder. The formulation may be used for the treatment of schizophrenia. The formulation may be used for the treatment of metabolic diseases. In particular, one can cite disorders of neurotransmitter metabolism and transport (Orphanet ORPHA:79169), and in particular disorders of biogenic amine metabolism and transport (Orphanet, ORPHA:79214) such as brain dopamine-serotonin vesicular transport disease. Of particular interest are tetrahydrobiopterin deficiencies (BH4 deficiencies) or any trouble of BH4 synthesis. One can thus cite guanosine triphosphate cyclohydrolase I (GTPCH) deficiency, 6-pyruvoyl tetrahydropterin synthase (PTPS) deficiency, pterin-4-alpha-carbinolamine dehydratase (PCD) deficiency and dihydropteridine reductase (DHPR) deficiency (also known as Segawa Syndrome or Dopamine-responsive dystonia (DRD) in the autosomal dominant form).

A method for treating a disease as listed above, including the step of administering a therapeutic dose of the suspension composition as disclosed to a patient in need thereof is also part of the invention. The invention thus features a method of treating a disorder. The method includes administering an effective amount of the suspension solution of any one of the above embodiments to a patient having the disorder. In some embodiments, the disorder is autism spectrum disorders, pervasive developmental disorders, schizophrenia, psychotic disorders, hereditary metabolic diseases, neurogenetic diseases, certain acquired diseases, Rett syndrome, epileptic encephalopathies, leukodystrophies, neuro-psychiatric disorders, post-anoxic myoclonies, "Lance and Adams syndrome," cerebellar ataxias, depression, suicidal thoughts, anxiety, alcoholism, fibromyalgia, insomnia, bulimia, or chronic headache. In some embodiments, the autism spectrum disorder is autism, Asperger syndrome, pervasive developmental disorder not otherwise specified, and childhood disintegrative disorder. In some embodiments, the autism spectrum disorder is autism.

The compositions herein disclosed result from extensive research by Applicant, with the aim to obtain a composition that can easily be dosed by the patient, and that has industrial and real-life application (in particular being stable for some time). The patient may thus reconstitute the suspension formulation by adding the buffer to the powdered formulation, and be able to take the daily appropriate dose of active ingredients. For compliance reasons, it is important that the patient does not have to reconstitute the dosage every day as this would be too burdensome for him.

In particular, due to the form of the formulation, the appropriate dose can be determined and adjusted by the patient himself. Consequently, the invention also discloses a method for adapting a therapeutic dose in a patient, comprising the steps of
(i) Administering a dose of the suspension composition as herein disclosed to the patient
(ii) Observing and/or assessing both the therapeutic effect on the patient and the presence of any side effect associated with the intake of 5-HTP, and
(iii) Increasing the dose to the patient if the therapeutic effect is not met or decreasing the dose in the presence of side effects.

Such method may be performed by a physician and/or by the patient himself (preferably under the control of the physician). Performance of the method is made possible by the specific form of the 5-HTP / carbidopa formulation.

One can also cite a method for treating a patient in need thereof, comprising the steps of
(i) Administering a dose of the suspension composition as herein disclosed to the patient,
(ii) Observing, or assessing the therapeutic effect on the patient and the presence of any side effect associated with the intake of 5-HTP, and
(iii) Providing a higher dose of the composition (increasing the volume) to the patient if the therapeutic effect is not met or providing a lower dose (decreasing the volume) in the presence of side effects (in particular that prove to exceed the therapeutic advantage gained by the patient).

In step (i), the dose is determined by the physician according to the disease of the patient (as seen above, the composition is able to be used in the treatment of various diseases and the doses can thus be adjusted depending on the disease and the patient). The dose chosen is supposed to have a therapeutic effect while minimizing the potential adverse effects. In step (iii), the physician shall increase the dose if there are no adverse effect and the therapeutic effect is not met or insufficiently met. The dose shall be decreased if side effects are observed, in particular if they are invalidating for the patient everyday life.

The composition according to the invention allows for tuning of the dose for a given disease or disorder, and a given patient. As an illustration, for autistic disorders, the dosage may be from 1 to 8 mg/kg/day of 5-HTP (thereby 0.25 to 2 mg/kg/day of carbidopa when a 4:1 ratio is used), with an increase in dosage if needed. The daily dosage may also be different (higher or lower) for other diseases or disorders. In particular, it may be advantageous to fractionate the taking of the suspension solution. As an illustration, one can provide 0.5 mg/kg three times a day in order to administer 1.5 mg/kg/day.

The invention also relates to a kit for preparing a suspension formulation, as disclosed above, comprising
i. A first container containing a predetermined amount of the powder composition (i.e. a blend of 5-HTP and carbidopa) as disclosed above, preferably with a bulking agent as disclosed above.
ii. A second container containing a predetermined amount of citrate buffer with a pH between 3.0 and 4.5. This second container, containing a liquid solution preferably also contains preservative and/or thickener agents.

It is preferred when the predetermined amount of citrate buffer is such that mixing of the content of the second container with the content of the first container leads to a suspension composition as disclosed above (50 mg/mL of active compounds, with a concentration of 40 mg/mL of 5-HTP and 10 mg/mL of carbidopa). Consequently, the ratio of 5-HTP and carbidopa are as disclosed above, and preferably 4:1.

In a specific embodiment, the kit further includes a dispensing device presenting graduation that provides a visual indication of individual dose or portions thereof. In one embodiment, the dispensing device is a syringe. In another embodiment, the dispensing device is a graduated medicine spoon. Such dispensing devices are known in the art.

Indeed, during the extensive research performed by Applicant to develop the composition herein disclosed, it was shown that a solution with only 5-HTP and Carbidopa could form lumps and sediment too quickly, and could thus not be convenient enough for the intended use. Such a solution would:
- Restrain manual reconstitution at home (magnetic stirring could be used to limit the lumps and obtain homogenous suspension, which would be acceptable for hospital use, but limit the use of the suspension for a patient at home), and
- Be limited by sedimentation, which could pose a problem to ensure that the proper dose is provided to the patient.

In order to help solve and minimize these issues, it may be advantageous to use thickeners and/or bulking agents.

### Pharmaceutical compositions

5-HTP and carbidopa are preferably formulated into pharmaceutical compositions for administration to individuals in a biologically compatible form suitable for oral administration *in vivo.* Accordingly, the present invention provides pharmaceutical compositions including 5-HTP and carbidopa, optionally in admixture with a suitable thickener, bulking agent, and/or preservative.

### Thickener

One or more thickening agents can be added in the solvent bottle to obtain a good suspension. This would also avoid fast sedimentation. A thickening agent or thickener is a substance which can increase the viscosity of a liquid without substantially changing its other properties. Any suitable thickener known in the art can be used, such as HPMC (hydroxypropyl methylcellulose such as Methocel® E4M Premium (the Dow Chemical Company), which does not complex with ionic species to form insoluble precipitates and presents a viscosity of about 2663-4970 mPa.s (2% in H₂O, 20°C)), agar, carrageenan, a gum (such as arabic gum, guar, xanthan, cellulose, Locust bean), a saccharide (such as carrageenan (preferably iota carrageenan), pullulan, konjac, and alginate). The preferred thickener is HPMC. The thickener is generally added at a concentration of 0.1-10.0% (w/v). In some embodiments, the thickener is added at a concentration of 0.5% (w/v), e.g., 0.1% (w/v), 1.0% (w/v), 2.0% (w/v), 5.0% (w/v), 7.0% (w/v), or 10.0% (w/v). It is preferred when the thickener is HPMC (in particular Methocel® E4M Premium) and added at a concentration of 0.5% (w/v).

Preferably, the thickener is added in the container containing the citrate buffer.

### Bulking agent

As indicated above, in order to avoid or limit the formation of lumps when mixing the solvent and the powder of 5-HTP and carbidopa, it may be useful to add a bulking agent into the container that stores the powder. This would also help to improve hydration of the thickener. Any suitable bulking agent can be used, for example, lactose, mannitol, sucrose, and mixtures thereof. Sucrose is preferred.

### Preservatives

A preservative or stabilizer may be added in order to, in particular, ensure good conservation of the liquid solution before addition to the powder composition, and to help stabilize carbidopa after the suspension solution has been reconstituted. This preservative is thus preferably added into the second container, containing the citrate buffer. Different preservatives are known in the art and any suitable preservative may be used in the invention. However, it is preferred to use sodium (Na) benzoate, which proved to be the best balance between preservation of the solution (including the suspension solution) and palatability.

### Chelating agent

In order to help stabilize the solution, one can also use a chelating agent. Such agents are known in the art, including, but not limited to, EDTA, and more specifically disodium EDTA.

It is to be noted that the presence of 0.5% of EDTA at pH 3.0 (which is a pH at which the compounds are stable and are not subject to degradation) leads to some crystallization of the products. Such crystallization disappears (or does not appear) when the pH is higher or the concentration is lower. However, increasing the pH decreases the stability of the active ingredients. Optimization of the pH (between 4.5 and 3.0, e.g., 4.0, or 3.5) and of the concentration of sodium EDTA (when used) makes it possible to both stabilize the products and avoid crystallization. A chelating agent, such as sodium EDTA, may be used at a concentration between 0.037 and 0.5% (w/v), e.g., between 0.050 and 0.5% (w/v), between 0.100 and 0.5% (w/v), between 0.150 and 0.5% (w/v), between 0.200 and 0.5% (w/v), between 0.250 and 0.5% (w/v), between 0.300 and 0.5% (w/v), between 0.350 and 0.5% (w/v), between 0.400 and 0.5% (w/v), or between 0.450 and 0.5% (w/v).

The invention also relates to a method for preserving a suspension composition as disclosed above, including the step of storing the composition at a temperature comprised between 2 and 8°C.

A preferred kit making it possible to obtain the suspension composition herein disclosed shall thus be in the form of:
- a first vial containing the active ingredients carbidopa and 5-HTP (e.g.
   at a ratio of 1:4) and a bulking agent (preferably sucrose, 1 part of active ingredients and 3 parts of bulking agent). Preferably, this vial does not contain any other ingredient.
- a second vial containing a liquid composition made with
   (a) citrate buffer (50 mM, pH between 3.0 and 4.5),
   (b) disodium EDTA (0.037% (corresponding to 1mM) to 0.5 % (w/V))
   (c) HPMC (0.3 to 0.8 % (w/V)), and
   (d) Sodium benzoate (0.2 to 0.5 % (w/V))

The amount of liquid in the second vial is adapted so that it can be poured in the first vial to obtain a suspension composition having the proper concentrations, as indicated above.

A specific kit includes:
- a first vial containing the active ingredients carbidopa and 5-HTP at a ratio of 1:4 and a bulking agent (preferably sucrose, 1 part of active ingredients and 3 parts of bulking agent), without any other element or excipient.
- a second vial containing a liquid composition made with
   (a) citrate buffer (50 mM, pH 3.0),
   (b) disodium EDTA (0.1% (w/V)
   (c) HPMC (0.5% (w/V)), and
   (d) Sodium benzoate (0.25% (w/V))

As indicated above, the HPMC is preferably Methocel® E4M Premium, or another Hydroxypropyl Methylcellulose with the viscosity as indicated above.

### Methods of treatment

Daily doses of 5-HTP may range between 50 and 250 mg (e.g., between 75 and 250 mg, between 100 and 250 mg, between 125 and 250 mg, between 150 and 250 mg, between 175 and 250 mg, between 200 and 250 mg, or between 225 and 250 mg). However, doses as low as 10 or 25 mg may be effective (e.g., 12 mg, 15 mg, 17 mg, 20 mg, or 22 mg). For some patients, higher doses may be needed, i.e., between 300-500 mg (e.g., between 325-500 mg, between 350-500 mg, between 375-500 mg, between 400-500 mg, between 425-500 mg, between 450-500 mg, or between 475-500 mg). Alternatively, the dosage amount can be calculated using the body weight of the patient. For example, the dose of 5-HTP, or a pharmaceutically acceptable salt thereof, administered to an patient may range from 0.5 to 8.0 mg/kg/day (e.g., 1.0 to 8.0 mg/kg/day, 1.5 to 8.0 mg/kg/day, 2.0 to 8.0 mg/kg/day, 2.5 to 8.0 mg/kg/day, 3.0 to 8.0 mg/kg/day, 3.5 to 8.0 mg/kg/day, 4.0 to 8.0 mg/kg/day, 4.5 to 8.0 mg/kg/day, 5.0 to 8.0 mg/kg/day, 5.5 to 8.0 mg/kg/day, 6.0 to 8.0 mg/kg/day, 6.5 to 8.0 mg/kg/day, 7.0 to 8.0 mg/kg/day, or 7.5 to 8.0 mg/kg/day). Administration may occur one or more times during a day, e.g., 1, 2, 3, or 4 times a day. Administration may also occur during the day, e.g., morning and/or afternoon, to provide treatment during waking hours. Alternatively, administration may occur one or more times during a week or month, e.g., weekly, biweekly, or monthly administration. In some embodiments, the administration is life-long. The reduction in symptoms of a serotonin-related disorder may be confirmed by patient, parent, caregiver, or health-care professional (e.g., a nurse) reports or may be confirmed by more detailed patient-reported outcome scales and/or neuropsychological batteries.

### EXAMPLES

### Example 1. Solubility of 5-HTP and carbidopa

The individual solubility of 5-HTP and carbidopa was assessed and compared to the data in the literature.

| **Solvent** | **Solubility (mg/mL)** | |
|---|---|---|
| | **5-HTP** | **Carbidopa** |
| Water | 1 - 10⁽¹⁾ | 1 - 10 ⁽²⁾ |
| 3N HCI | - | 100 - 1000 ⁽³⁾ |
| Methanol | - | 1 - 10 ⁽³⁾ |
| Ethanol | - | 0.1 - 1 ⁽²⁾ |
| Acetone | - | < 0.1 ⁽³⁾ |
| Methylene chloride | - | < 0.1 ⁽³⁾ |
| Chloroform | - | < 0.1 ⁽²⁾ |
| Ether | - | < 0.1 ⁽³⁾ |

| | | |
|---|---|---|
| -: Not available. Sources: (1) ChemicalBook.com (2) Ph. Eur. (European Pharmacopeia) (3) Carbidopa Certificate of Analysis of manufacturer | | |

**Table 1. Solubility of 5-HTP and carbidopa from literature**

| **Solvent** | **UV-measured solubility (mg/mL)** | |
|---|---|---|
| | **5-HTP** | **Carbidopa** |
| **Aqueous** | | |
| 0.1N HCI | 29.4 | 11.8 |
| Phosphate buffer, pH5 | 8.4 | 1.5 |
| Phosphate buffer, pH6.8 | 8.9 | 2.1 |
| Phosphate buffer, pH9 | 9.3 | 4.7 |

| **Cosolvents** | | |
|---|---|---|
| Ethanol | 5.1 | 0.07 |
| Propylene glycol | 35.4 | 15.0 |
| PEG400 | 2.3 | 1.5 |
| Transcutol HP | 13.7 | 0.6 |

| **Tensides** | | |
|---|---|---|
| Polysorbate 20 (5% in water) | 9.4 | 1.4 |
| Kolliphor RH40 (5% in water) | 9.5 | 1.5 |
| Solutol HS15 (5% in water) | 9.0 | 1.4 |
| Sepiclear (5% in water) | 14.5 | 3.1 |

| **Polymers** | | |
|---|---|---|
| 5% Soluplus in water | 12.4 | 12.9 |

| **Complexing agents** | | |
|---|---|---|
| 20% w/v HP-β-cydodextrin in water (Kleptose) | 17.0 | 3.1 |
| 10% w/v M-β-cyclodextrin in water (Kleptose crysmeb) | 13.5 | 2.8 |

| **Lipids** | | |
|---|---|---|
| Sunflower oil | < 10 ⁽¹⁾ | < 10 ⁽¹⁾ |
| Labrasol ALF | 4.8 | 12.8 |
| Labrafac lipophile | < 10 ⁽¹⁾ | < 10 ⁽¹⁾ |

| | | |
|---|---|---|
| ⁽¹⁾ Accurate measurement not possible due to diverging spectrum. | | |

### Table 2. Solubility results

### Results

The obtained solubility results confirm the data for aqueous solubility found in the literature. The solubility increases upon acidifying the water to pH 1 for both compounds. Addition of surfactants does not significantly increase the solubility Complexation with HP-β-CD increases the solubility for 5-HTP, but has no effect on carbidopa. The highest solubility was observed in propylene glycol, for both compounds. The other co-solvents tested did not show improved solubility.

Based on the outcome of these solubility tests, a second wave of solubility testing was performed, with acidified vehicles and combinations thereof.

The highest solubility was obtained in 40% (w/v) HP-3-cyclodextrin + 38.4 mg/mL citric acid, pH2.0, for both APIs (Active Pharmaceutical Ingredients). The marginal increase in solubility in this vehicle, compared to similar solution containing only 20% (w/v) HP-3-cyclodextrin is only -20% for 5-HTP and -40% for carbidopa. Therefore, and since dosing of HP-3-cyclodextrin is limited to about 8 - 16 g/day (for max. 14 days, short-term Phase I studies), it was foreseen to use the vehicle containing only 20% HP-b-cyclodextrin as the first choice for dosing safety reasons, *i.e.:*
- for a 600 mg 5-HTP/60 mg carbidopa dose with a 30 mg/mL 5-HTP/3 mg/mL carbidopa solution in solvent A (40% HP-β-CD), 20 mL solution is required, corresponding with an intake of 8 g HP-β-CD, and;
- for a 600 mg 5-HTP/60 mg carbidopa dose with a 30 mg/mL 5-HTP/3 mg/mL carbidopa solution in solvent B (20% HP-β-CD), 20 mL solution is required, corresponding with an intake of only 4 g HP-β-CD.

### Example 2. Screening stress test study

Stress studies were performed on the separated reference solutions and a mix solution under heat (60°C for 24 h), and under acidic, basic, or oxidative (H₂O₂) conditions, and ICH light (700 W/m² for 8 h) conditions.

It was found that carbidopa is very unstable in solution and is quickly degraded.

The only condition which did not influence carbidopa (i.e., caused minimal carbidopa degradation) is oxidative stress. At T0 and T24h, the assay (% I.c.) remains as high as the control at T0.

Further, it was found that heat had a very high influence on carbidopa as it led to 90% degradation. Light also led to about 88% degradation after 8 h of exposure. Acidic conditions immediately led to some carbidopa degradation (i.e., more than 20%), with no further degradation after 24 h. Influence of basic medium led to degradation (i.e., about 30%) that was noticeable after 24 h.

The same observations were made for carbidopa in solution in presence of 5-HTP. It was found that 5-HTP was not sensitive to heat, acidic and oxidative stress. A slight influence of basic medium and light was observed after 24 h (i.e., about 15% degradation).

It was found that 5-HTP was not sensitive to heat, acidic and oxidative stress. A slight influence of basic medium and light was observed after 24h (about 15% degradation).

When 5-HTP was present in solution with carbidopa, mainly light and acidic stress influenced the degradation of 5-HTP. Other stress conditions did not cause a significant degradation of 5-HTP.

This suggests an interaction between both compounds.

### Example 3. Stability of 5-HTP - carbidopa formulations

Compositions including 5-HTP / carbidopa (4:1 w/w) in various solvents were submitted to a stability study for 2 weeks or 1 month, at 5°C and 40°C/75%RH (relative humidity).
- 40% HP-β-CD + 38.4 mg/mL citric acid pH 2
- 0.5% HPMC
- 0.5% HPMC + 0.1% ascorbic acid (pH 4)
- 0.5% HPMC + 0.1% ascorbic acid (pH 2)
- 5% kolliphor P407
- 5% kolliphor P407 + 0.1% ascorbic acid (pH 4)
- 5% kolliphor P407 + 0.1% ascorbic acid (pH 2)
- H₂O

The results of this accelerated stability test (40°C, 75% RH) were not very satisfying. Impurities mainly caused by the degradation of carbidopa were detected. The metabolites from 5-HTP were below the detection limit. The results of the stability assay at 5°C showed presence of impurities caused by degradation of carbidopa.

### Example 4. Determination of appropriate formulations

Various formulations containing
- Different ratios of 5-HTP:carbidopa (1:1; 4:1; 5:1)
- Different pH of citrate buffer at 50mM (pH 5.3; pH 4.5; pH 3.0) were tested.

The solution was reconstituted by adding a solvent liquid solution including the citrate buffer in a vial including the powdered 5-HTP and carbidopa. Disodium EDTA, HPMC, and sodium benzoate were also present in solution. In some solutions, potassium benzoate was also added. After 7 days of storage at 5°C and 25°C/60% RH, it was noted that the pH of the compositions remained stable (initial citrate buffer pH±10%). The viscosity also remained within the expectations. The composition that provided the best results (i.e., maintained the suspension appearance, had lower degradation of the active ingredients, and in particular carbidopa) was the composition with a 5-HTP:carbidopa ratio of 4:1 and citrate buffer pH 3.0.

For the other compositions, the appearance of the solution changed from an initial white homogenous suspension to a slightly brown, homogenous suspension. It was also noticed that degradation of carbidopa increased when the pH increased. It was noted that using a 1:1 ratio at pH 3.0 also made it possible to limit the degradation of carbidopa, but that this induced some 5-HTP degradation. Furthermore, the appearance of the formulation changed over time. This formulation may nevertheless be used as the degradation levels remained low.

### Using the 5-HTP/Carbidopa (ratio 4/1) - Citrate buffer pH 3.0 (with EDTA):

- Assay 5-HTP: stable up to 7 days of storage at 5°C and 25°C/60% RH.
- Assay carbidopa: stable up to 7 days of storage at 5°C and 25°C/60% RH.
- No degradation products of 5-HTP were observed.
- The carbidopa-related degradation products are relatively stable when stored at 5°C and their presence was slightly increased after 3 days of storage at 25°C/60% RH and
- appearance after reconstitution: no change was observed at 5°C and 25°C/60% RH.

### Example 5. Conclusion

Taken together, these results show that being able to obtain a liquid (and hence easily dosable) suspension of 5-HTP and carbidopa required extensive development of an appropriate formulation, including the choices of buffers, adjuvants and active drugs ratio.

In order to make it possible for a patient to use this formulation, and in view of the low stability of the active ingredients in water solution, it is necessary to provide a powdered solution containing the active ingredients, that is to be suspended (5-HTP will dissolve whereas carbidopa will remain in the form of particles in a suspension) in a solution of citrate buffer at a low pH, comprised between 3.0 and 4.5. In particular, a 50mM citrate buffer solution at pH 3.0 is adequate. As indicated above, EDTA is to be used in a quantity sufficient enough to avoid precipitation of the suspension (generally less than 0.5% at pH 3.0).

The ratio of 5-HTP/carbidopa was such that the more carbidopa is present relatively to 5-HTP, the less carbidopa degradation occurs, however the more 5-HTP degradation is observed.

A 5-HTP/carbidopa ratio from 1:1 to 4:1 is thus perfectly suitable, depending on the pH in order to minimize carbidopa and 5-HTP degradation.

Such reconstituted solutions can be stored for a week in the refrigerator (around 4°C) without degradation of the ingredients. The formulation prior to reconstitution makes it possible to handle and store the two containers at room temperature for some time without degradation of the products.

## Claims

1. A pharmaceutical composition comprising 5-HTP and carbidopa, wherein said composition is in the form of a powder.

2. The pharmaceutical composition according to claim 1, wherein the ratio of 5-HTP to carbidopa is between 5:1 and 1:1 by weight, preferably 4:1 by weight.

3. The pharmaceutical composition of claim 1 or 2, further comprising a bulking agent, preferably selected from the group consisting of lactose, mannitol, sucrose and mixtures thereof.

4. The pharmaceutical composition of any one of claims 1 to 3, wherein the 5-HTP and the carbidopa are the only active ingredients in the composition.

5. The pharmaceutical composition of any one of claims 1 to 4, only containing 5-HTP, carbidopa and a bulking agent.

6. A suspension solution comprising 5-HTP and carbidopa in citrate buffer solution, wherein the pH of the solution is comprised between 3.0 and 4.5.

7. The suspension solution of claim 6, wherein the citrate buffer is a 50mM citrate buffer at pH 3.0.

8. The suspension solution of claim 6 or 7, wherein 5-HTP is solubilized and carbidopa is in the form of particles in suspension.

9. The suspension solution of any one of claims 6 to 8, wherein the concentration of 5-HTP in the solution is comprised between 10 and 100 g/L.

10. The suspension solution of any one of claims 6 to 9, wherein the concentration of carbidopa in the solution is comprised between 2.5 and 25 g/L.

11. The suspension solution of any one of claims 6 to 10, susceptible to be obtained by addition of a citrate buffer solution to the pharmaceutical composition of any one of claims 1 to 5.

12. A method for preparing a suspension solution of carbidopa and 5-HTP, comprising the step of adding a citrate buffer solution with a pH comprised between 3.0 and 4.5 to the pharmaceutical composition of any one of claims 1 to 5.

13. The method of claim 12, wherein the citrate buffer solution further comprises a thickening agent, a preservative, or a chelating agent.

14. The method of claim 12 or 13, wherein the citrate buffer solution further comprises a thickening agent which is an hydroxypropyl methylcellulose.

15. The method of any one of claims 12 to 14, wherein the citrate buffer solution further comprises a preservative which is sodium benzoate.

16. The method of any one of claims 12 to 14, wherein the citrate buffer solution further comprises a chelating agent which is sodium EDTA.

17. The method of any one of claims 12 to 16, wherein the concentration of 5-HTP is comprised between 10 and 100 mg/mL in the suspension composition.

18. The suspension solution of any one of claims 6 to 17 as a medicament.

19. The suspension solution of any one of claims 6 to 18 for its use in the treatment of a disease or disorder where increase of serotonin is required, in particular for the treatment of a disorder selected from an autistic spectrum disorder, a disorder of biogenic amine metabolism and transport, a disorder of neurotransmitter metabolism and transport, a brain dopamine-serotonin vesicular transport disease, a tetrahydrobiopterin deficiency (BH4 deficiency), a trouble of BH4 synthesis, a guanosine triphosphate cyclohydrolase I (GTPCH) deficiency, a 6-pyruvoyl tetrahydropterin synthase (PTPS) deficiency, a pterin-4-alpha-carbinolamine dehydratase (PCD) deficiency and a dihydropteridine reductase (DHPR) deficiency.

20. A kit for dispensing a pre-determined dose of carbidopa and 5-HTP to a patient comprising
i. a first container comprising a predetermined amount of the pharmaceutical composition according to any one of claims 1 to 5,
ii. a second container comprising a predetermined amount of citrate buffer, with a pH between 3.0 and 4.5, and optionally
iii. a dispensing device presenting graduation that provide a visual indication of individual dose or portions thereof.

21. The kit of claim 20, wherein
- the first container contains a powder composition made with
(a) active ingredients: carbidopa and 5-HTP at a 1:4 ratio, and
(b) a bulking agent, preferably sucrose, at a ratio of 1 part of active ingredients for 3 parts of bulking agent, and
- the second container contains a liquid composition made with
(a) citrate buffer 50 mM, at a pH comprised between 3.0 and 4.5,
(b) disodium EDTA at a concentration between 0.037 and 0.5 % (w/v)
(c) hydroxypropyl methylcellulose at a concentration between 0.3 and 0.8 % (w/v), and
(d) sodium benzoate at a concentration between 0.2 and 0.5 % (w/v).
